Europäisches Patentamt

European Patent Office

Office européen des brevets

(11) Publication number: **0 484 400 B1**

(12) **EUROPEAN PATENT SPECIFICATION**

(45) Date of publication of patent specification: **22.03.95**

(51) Int. Cl.[6]: **C07C 51/41**, C07C 53/126, C07C 53/128, C07C 57/03, C07C 57/12

(21) Application number: **90911524.8**

(22) Date of filing: **24.07.90**

(86) International application number:
**PCT/US90/04136**

(87) International publication number:
**WO 91/01962 (21.02.91 91/05)**

(54) **CONTINUOUS PROCESS FOR PREPARATION OF AOUEOUS DISPERSIONS OF METAL SOAPS.**

(30) Priority: **26.07.89 US 385754**

(43) Date of publication of application:
**13.05.92 Bulletin 92/20**

(45) Publication of the grant of the patent:
**22.03.95 Bulletin 95/12**

(84) Designated Contracting States:
**AT BE CH DE ES FR GB IT LI NL SE**

(56) References cited:
**GB-A- 1 053 805**
**GB-A- 1 074 093**

**PATENT ABSTRACTS OF JAPAN, vol. 11, no. 232 (C-437)(2679), 29 July 1987, & JP-A-6245551**

(73) Proprietor: **HENKEL CORPORATION (a Delaware corp.)**
**300 Brookside Avenue**
**Ambler, PA 19002 (US)**

(72) Inventor: **KOENIG, H., Steve**
**2613 Giverny Drive**
**Charlotte, NC 28226 (US)**
Inventor: **SPEENBURGH, Gary, L.**
**231 Kingston Road**
**Parsippany, NJ 07054 (US)**

(74) Representative: **von Kreisler, Alek,**
**Dipl.-Chem. et al**
**Patentanwälte**
**von Kreisler-Selting-Werner**
**Postfach 10 22 41**
**D-50462 Köln (DE)**

**Description**

The invention is a continuous process for the production of aqueous dispersions of insoluble metal salts of organic acids (herein after noted as metal soaps) and particularly insoluble metal salts of higher fatty acids. The dispersions of the metal soaps can be utilized as prepared or they can be dried to produce finely divided particulate metal soaps.

Background of the Invention:

1. Metal soaps have found many uses in industry. They generally possess many of the desirable properties of the acids from which the metal soaps are manufactured. Some applications of the metal soaps require that they be prepared in a form of a stable aqueous dispersion. The aqueous dispersions generally comprise the metal soap, water and dispersing agents for the metal soap. The dispersing agent is generally a surfactant or mixture of surfactants which are generally of the anionic and the nonionic type.

DESCRIPTION OF RELATED ART

Metal soap dispersions are available commerically at solid contents of from about 35 to about 75 percent by weight. The aqueous dispersions typically contain about 30 to about 60 percent metal soap, the remaining solids comprising emulsifiers, dispersion aids and other processing aids. The additives are included to enhance the production process for the metal soap or the end-use of the product.

Aqueous dispersions of metal soaps have been prepared in the past by three methods.

One of the oldest methods comprises reacting the organic acid with an oxide or hydroxide of the metal, in the presence of a small amount of water, to form a solid metal soap of the acid, grinding the metal soap and dispersing the ground material in water by means of a dispersing agent.

More recent processes prepare the dispersion of the metal soap by reacting a fatty acid with an oxide or hydroxide of the metal in the presence of a dispersing agent and the water in which the metal soap of the fatty acid is to be dispersed. The processes are generally batch processes and require several hours to complete the reaction. The dispersions prepared by the known methods generally require that the dispersion be milled to provide a stable dispersion having particles within a required particle size range.

The metal soaps have been prepared by a double decomposition method wherein the metal soap is precipitated from an aqueous solution by reaction of an alkali metal salt of the fatty acid with an acid salt of the metal used to form the metal soap. The metal soap precipitates as a fluffy material, can be recovered from the aqueous medium washed free of the alkali metal salts then dispersed in an aqueous medium to form a dispersion. The known processes are generally batch processes and require milling of the dispersion to provide a stable product having dispersed therein metal soap of a small particle size.

There are many patents which disclose processes for preparing metal soaps and dispersions of metal soaps.

Patents such as U.S.-A-2,660,568, U.S.-A-3,803,188, U.S.-A-4,060, 535, U.S.-A-4,307,027, Japanese Kokai 51/34904 (March 25, 1976), Kokai 54/8606 (January 23, 1979), Kokai 59/51236 (March 24, 1984) disclose processes for preparing metal soaps and aqueous dispersions of metal soaps. In particular U.S-A-3,803,188 discloses the three general methods used to prepare metal soaps.

A batch process is disclosed in East German Patent DD-A-106629, Patented June 20, 1974. The patent discloses a process for making dispersions of metal soaps such as calcium, zinc or lead stearate. The process comprises preparing a mixture of water, a dispersing agent and the hydroxide of the metal with acetic acid at a temperature above the melting point of the fatty acid in a Cowles Disolver and introducing the fatty acid into the mixture at a temperature above its melting point.

A continuous process for preparing dispersions of metal soaps is disclosed in East German Patent DD-A-241900. The process discloses continuously introducing a suspension of the metal oxide or metal hydroxide into a cascade of stirred reactors. The fatty acid is introduced into each of the stirred reactors depending upon the concentration of the fatty acid at that point of the process. The process is relatively complex in that the concentration of the fatty acid in a plurality of reactors must be monitored and the addition of the fatty acid into each of the series of reactors must be controlled. The process provides a relatively long residence time of the dispersion in the system.

JP-A-59 051 236 (Chemical Abstracts CA 101 : 40223k) discloses that a dispersion of a metal soap can be prepared by grinding a metal soap with water and a dispersing agent in a media mill which is known to be a good grinding tool for pigments and other materials which must be provided as a fine dispersion.

According to this disclosure the metal soap was ground to form the dispersion in the presence of water and dispersing agents for a period of 15 minutes.

BRIEF DESCRIPTION OF THE INVENTION

The invention is a continuous single step process for the production of aqueous, metal soap dispersions which comprises continuously introducing an aqueous suspension of an oxide or hydroxide of the metal, at least one dispersing agent and a molten organic acid into a media mill and removing a dispersion of the metal soap from the media mill. The process of the present invention is carried out at a residence time in the range of 1 to 55 minutes and produces a dispersion having a low-free fatty acid content. The process does not require additional milling to provide an aqueous dispersion. The dispersion is stable has a small particle size and substantially no oversized material.

In detail the present invention relates to a continuous process for preparing an aqueous dispersion of a metal soap which comprises: forming a mixture by continuously introducing at least one carboxylic acid having from 6 to 30 carbon atoms, at least one oxide or hydroxide of a metal, which forms a metal soap with the at least one carboxylic acid, water and at least one dispersing agent into a media mill, wherein water comprises at least 25% by weight of the mixture and reacting the mixture in the media mill for from 1 to 55 minutes to form an aqueous dispersion of the metal soap which comprises from 25% to 65% water.

BRIEF DESCRIPTION OF THE DRAWINGS

Fig. 1 is a schematic representation of a typical prior art process for preparing aqueous dispersions of metal soaps.

Fig. 2 is a schematic representation of the process of the present invention.

DETAILED DESCRIPTION OF THE INVENTION

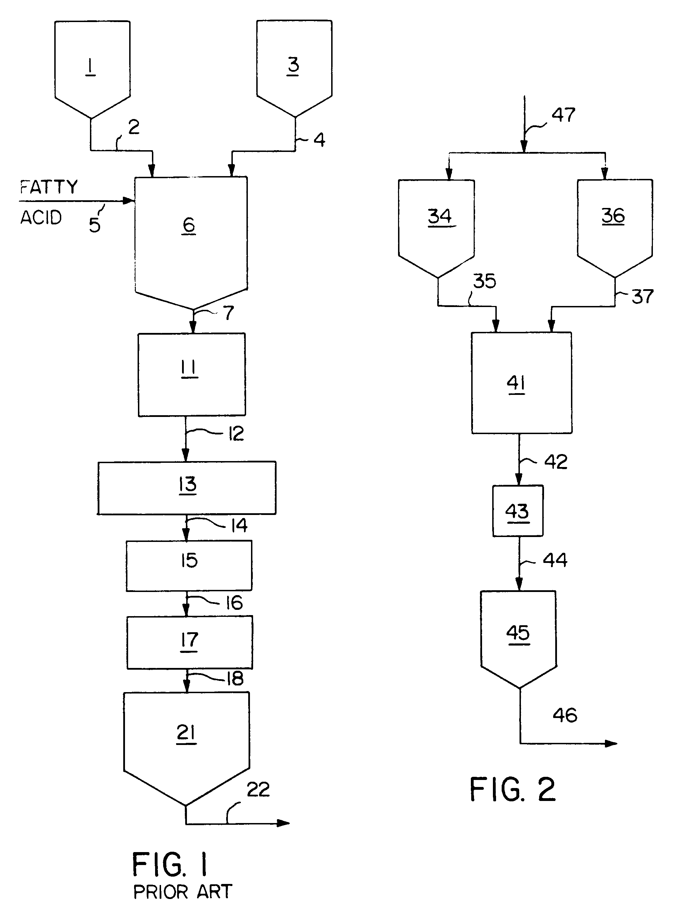

The advantages of the process of the present invention can be better understood by comparison with a batch process which is known in the prior art. Fig. 1 is a schematic representation of a prior art batch process for preparing a calcium fatty acid soap dispersion. A dispersing agent or mixture of dispersing agents is prepared in tank 1 and introduced into the reactor 6 through line 2. A suspension of lime in water is prepared in tank 3 and introduced into reactor 6 through line 4. Reactor 6 is a well agitated reactor. After the dispersing agent and the lime suspension had been introduced into reactor 6, the aqueous mixture is heated to a temperature near the melting point of the fatty acid. Molten fatty acid is then introduced through line 5 into the agitated suspension of the calcium hydroxide and the agitation continued until the fatty acid is substantially reacted to the desired metal soap. The reaction generally requires 1 to 10 hours to complete. The temperature in the reactor is controlled by cooling means in the reactor jacket.

After the reaction is completed, the dispersion is passed from the reactor 6 through line 7 to holding tank 11. Holding tank 11 is necessary to permit the reactor to again be filled with reactants and another batch of the dispersion of the metal soap prepared. The dispersion from holding tank 11 is then introduced through line 12 into a milling or grinding means 13. The grinding means 13 can comprise one or more grinding means which reduces the particle size of the metal soap dispersion to the required particle size range. Suitable grinding means are known in the art. The ground dispersion then passes through line 14 through heat exchanger 15, and line 16 to size separating means 17. In size separating means 17 the oversize particles in the dispersion are separated from the dispersion and can be returned to the grinding means. The dispersion of the required particle size passing through size separating means 17 passes through line 18 to production holding tank 21. The dispersion is mixed in holding tank 21, sampled and if it meets specifications can be transferred to a storage tank (not shown) through line 22 or transferred for use.

As can be seen from the schematic diagram of a conventional process, the process is a batch one which requires reacting and milling steps and requires a substantial amount of process equipment to accomplish the process.

In contrast to the conventional processes for preparing metal soap dispersions, Fig. 2 is a schematic representation of the process of the present invention.

As shown in Fig. 2, a dispersing agent or a mixture of dispersing agents is introduced into the fatty acid in feed tank 34 and into the metal oxide or hydroxide aqueous suspension in tank 36 through line 47 and mixed therewith. The fatty acid, in fatty acid holding tank 34, is in a molten condition. The molten fatty acid is continuosly introduced into the reactor 41 through line 35 and the aqueous suspension of the metal oxide or hydroxide in holding tank 36 is continuously introduced through line 37 into reactor 41.

Reactor 41 is a media mill. The temperature in the media mill can be controlled by circulating cooling fluid in the jacket or internally arranged cooling means. In the mill, a media is caused to move about and contact the particles of the fatty acid and the metal oxide or hydroxide to cause them to react and to provide dispersed particles in a suitable particle size range.

The residence time in the media mill is relatively short and ranges from 1 to less than 55 minutes. Preferably from 2 to 20 minutes. The dispersion then passes from the media mill 41 through line 42 through heat exchanger 43 and line 44 to holding tank 45. In holding tank 45, the dispersion is sampled to determine if it meets the proper particle size, viscosity, free acid and other specifications and is then passed to a storage tank (not shown) through line 46.

The particle size at the outlet of the media mill reactor is uniform and generally no screening or sizing operation is required. However, as a safety measure, should something malfunction in the system, a sizing means can be introduced into line 44 between heat exchanger 43 and holding tank 45.

As can be seen from a comparison of the schematic of the process of the present invention and the schematic for the batch process, the process of the present invention is simpler, requires less equipment and can be operated at a lower cost than the known processes.

Soaps of metals such as aluminum, barium, calcium, cadmium, cobalt, copper, iron, lead, lithium, magnesium, manganese, nickel, strontium and zinc can be prepared by the process of the present invention. Generally, the process of the present invention can be adapted to provide dispersions of the metal soaps which can be prepared by the batch processes of the prior art.

The fatty acids which can be utilized to prepare metal soap dispersions, by the process of the present invention generally contain from 8 to 30 and preferably 10 to 24 carbon atoms. The acids can be saturated or unsaturated, straight chain or branched chain. Dispersions of metal soaps of fatty acids such as octanoic acid, nonanoic acid, decanoic acid, undecanoic acid, dodecanoic acid, tridecanoic acid, tetradecanoic acid, pentadecanoic acid, hexadecanoic acid, heptadecanoic acid, octadecanoic acid, nonadecanoic acid, eicosanoic acid, hencosanoic acid, docosanoic acid, and their isomers can be prepared by the process of the invention. Dispersions of soaps of the unsaturated fatty acids such as the octenoic acids, nonenoic acids, decenoic acids, undecenoic acids, dodecenoic acids, tridecenoic acids, tetradecenoic acids, pentadecenoic acids, hexadecenoic acids, heptadecenoic acids, octadecenoic acides, nonadecenoic acids, eicosenoic acids, docosenoic acids, tetracosenoic acids, and their isomers can be prepared by the process of the evention. Dispersions of metal soaps of dienoic, trienoic and, tetraenoic acids having from 8 to 24 carbon atoms can be prepared by the process of the invention.

Dispersions of metal soaps of hydroxy substituted fatty acids having from about 8 to about 30 carbon atoms can be prepared by the process of the invention. Dispersions of water insoluble soaps of alicyclic and aromatic acids can also be prepared by the process of the invention.

Useful surfactants or dispersing agents include anoinic surfactants such as ethoxylated alkyl phenol sulfates and sulfonates, fatty acid sulfates and sulfonates, petroleum sulfates and sulfonates, alcohol sulfates, ethoxylated alcohol sulfates, fatty acid ester sulfates, alkyl benzene sulfonates, alkyl napthalene sulfonates, sulfosuccinates, taurates, betaines and carboxylated alcohol ethoxylates. Nonionic surfactans such as ethoxylated alcohols, ethoxylated alkyl phenols, ethoxylated amines or amides, ethoxylated fatty acid esters, propylene oxide-ethylene oxide, random and block copolymers, fatty esters and polyethylene glycol ethers can be used as dispersing agents in the practice of the invention.

The dispersing agents are present in a sufficient amount to provide a stable dispersion. Generally, the dispersing agent or mixture of dispersing agents is in the range of from 0.5 to 20% by weight of the dispersion and preferably in the range of 1.5 to 10% by weight of the dispersion. The amount and type of dispersing agent is not critical except that it must be capable of providing a stable dispersions. Special user requirements may require the use of special dispersing agents.

The process can also be utilized to prepare dispersions of metal soaps from aromatic and alicyclic acids. The process can be utilized to prepare metal soaps of aromatic acids such as naphthenic acid. Process of the present invention can be generally utilized to prepare dispersions of metal salts of acids which provide water insoluble metal salts (metal soaps).

The dispersing agents used in the process of the present invention are conventional and well known in the art. Both anionic and nonionic surfactants can be used to prepare aqueous dispersions of metal soaps. Mixtures of anionic and nonionic surfactants are preferred since the advantageous properties of each of the surfactants is retained in the mixture. However, some applications require special dispersing agents to meet special end use requirements.

In the process of the present invention, the dispersing agents can be added to the aqueous suspension of the metal hydroxide or oxide, can be added to the molten fatty acid, can be added to each of the reactants or can be added directly into the reactor along with the suspension of the metal oxide or hydroxide in water and the fatty acid. However, it is preferred that the dispersing agents be introduced in admixture with at least one of the suspension of the metal oxide or hydroxide in water or with the molten fatty acid. The premixing of the dispersing agent with the reactants permits the introduction of the dispersing agent into the reactor in a metered amount without the requirement that an additional stream of reactants be metered into the reactor. For special requirements, additional dispersing agents or surfactants can be introduced into the dispersion leaving the media mill reaction zone. However, at least part of the dispersing agent should be introduced into the media mill reactor alone or preferably mixed with at least one of the fatty acid or the metal hydroxide or oxide dispersion.

The process of the present invention generally uses about a stoichiometric amount of multivalent metal compound to neutralize the fatty acid. A small excess or deficiency of metal hydroxide or oxide can be used to meet user requirements. Generally a small excess of 1 to 10% on an equivalent basis of the metal hydroxide or oxide is used. The dispersion generally contain from 25% to 65% by weight water preferably from 30 to 55% by weight water based on the weight of the dispersion.

The process can be operated over a broad temperature range. A temperature of from 25-100°C can be used. However a temperature in the range of 35°C to 95°C is preferred and most preferred is temperatures in the range of 50°C to 95°C. Temperatures above 100°C can be used but require special pressure equipment and product handling. Temperatures in the range of 50°C to 95°C provide for a rapid reaction and produce a dispersion with low fatty acid content. Higher temperatures are not required. The reaction temperature does not have to be above the melting point of the acid or soap.

The media mill utilized as the reaction zone in the process of the present invention comprises a vessel containing particulate material which tumble or move in the vessel. The particulate material can be moved in the vessel interior by rotating or vibrating the vessel or by providing means which stir the media. When the media or particulate material is moved by vibrating or stirring, the vessel can be arranged horizontaly or vertically. Due to the flexibility in arranging the feed introduction and product discharge means, a horizontally arranged media mill reaction zone is preferred.

Media mill as used herein refers to grinding and milling devices which utilize the movement of solid particles known as media within the aparatus to impart energy to the material being milled. Movement of the media can be achieved by rotating or vibrating the vessel containing the media. Movement of the media can also be provided by stirring means provided in the vessel or reaction zone. A combination of stirring and vessel movement can also be used.

In a preferred embodiment, a horizontaly arranged media mill manufactured by Premier Mill Corporation, charged with glass or ceramic beads is used as the reaction zone for the process of the invention.

The mill is charged with media, with a particle size in the range of from 1 to 3 mm in diameter. The media can be glass, ceramic or metal particles or the like. The process of the present invention has been found to achieve excellent results utilizing media comprising glass beads of 1.5-2.5 mm in diameter.

The void volume in the mill is generally filled from 50 to 95 percent with the grinding media. Preferably the mill is charged with from 62 to 90 percent of the void volume with the grinding media.

A preferred aparatus, the Premier Mill comprises a horizontally arranged vessel with a rotating means for stirring the media. The rotating means generally rotates at from 1000 to 3000 revolutions per minute. Lower or higher rotation speeds may be utilized and is dependent upon the media being used, and the size of the aparatus. The rotating device contacts the media and causes the media to move within the reaction zone. The impact of the media with the particles of the fatty acid and the metal oxide or hydroxide and the precipitated metals soap causes the reaction to proceed at a rapid rate and to produce a dispersion with a small particle size.

The following examples are presented to illustrate the process of the invention. The examples are for illustration only and are not intended to be a limitation to the invention. The process is illustrated by an embodiment for production of calcium stearate but other metal soaps can be prepared by the process.

In the examples, a suspension of calcium hydroxide in the water for the dispersion was prepared. A portion of the dispersing agents was added to the suspension. The suspension of calcium hydroxide was maintained at 20 to 30 degrees centigrade.

A portion of the dispersing agent was mixed with molten stearic acid at a temperature of 80 to 85 degrees centigrade.

The reaction zone was a horizontally arranged Premier Super Mill™ with a free volume of 15 liters charged with 1.5 or 2 millimeter diameter glass beads or 1.7 or 2mm diameter zirconium silicate beads or 1.7mm zirconium oxide beads. The mill comprised a shell with a removable end closure which carried a rotating milling shaft. The milling shaft carried discs which when rotated imparted energy and movement to the beads. The rate of rotation of the milling shaft was varied from 1100 to 1650 r.p.m.

The mill was heated to 50 to 55 °C by passing hot water through the mill. When the mill temperature reached 50 to 55° centigrade, the flow of hot water was stopped, the milling shaft was started rotating and the flow of calcium hydroxide suspension and molten fatty acid was started into the mill. The temperature of the dispersion leaving the mill was controlled by flow of cooling water through the mill shell.

The examples show the composition of the feed streams, the feed rate, retention time, and product temperature. The table presents a summary of the data and the properties of the dispersions. The examples were carried out according to the process illustrated in Fig. 2.

Example 1

Lime Slurry Feed

318.00 kg (702 Lbs) Water
41.67 kg (92 Lbs) Hydrated Lime (MV300/Mississippi Lime Co.)
1.13 kg (2.5 Lbs) Ethoxylated Nonyl Phenol (Hyonic® PE-110/Henkel Corp.)
1.81 kg (4.0 Lbs) sodium salt of an Alkyl Aryl Sulfonate (Witconate® 1238/Witco)
Agitation was started and the temperature adjusted to 20-30°C.

Fatty Acid Feed

294.45 kg (650 Lbs) $C_{18}$ fatty acid (Industrene® 7018/Witco Chemical)
16.76 kg (37 Lbs) Ethoxylated Nonyl Phenol (Hyonic® PE-100/Henkel Corp.)
1.58 kg (3.5 Lbs) $C_{36}$-$C_{54}$ dimer/trimer fatty acid (Empol® 1022/Emery Chemicals)
The vessel was heated until the charge was molten. Agitation was started and the temperature adjusted to 80-85°C.

The Premier Mill was charged with 2mm glass beads to 90% of the available volume. The mill was preheated to 55°C by passing hot water through the mill chamber. Simultaneously, the mill shaft was started at 1650 RPM, lime slurry feed was started at 0.43 kg/min (0.96 lb/min.), and molten fatty acid feed was started at 0.34 kg/min (0.76 lb/min.) rate. The average retention time (ART) in the mill was 8.9 min. The temperature of the exiting material was controlled at 70°C by cooling water applied to the jacket of the mill. The product was cooled to room temperature.

Example 2

Lime Slurry Feed

339.75 kg (750 Lbs) Water
41.67 kg (92 Lbs) Hydrated Lime (MV300/Mississippi Lime Co.)
1.13 kg (2.5 Lbs) Ethoxylated Nonyl Phenol (Hyonic® PE-100/Henkel Corp.)
1.81 kg (4.0 Lbs) sodium salt of an Alkyl Aryl Sulfonate (Witconate® 1238/Witco)
21.74 kg (48 Lbs) Peg 600 Monolaurate (Nopalcol® 6-L/Henkel Corp.)
Agitation was started and the temperature adjusted to 20-30°C.

Fatty Acid Feed

294.45 kg (650 Lbs) $C_{18}$ fatty acid (Industrene® 7018/Witco Chemical)
16.76 kg (37 Lbs) Ethoxylated Nonyl Phenol (Hyonic® PE-100/Henkel Corp.)
1.58 kg (3.5 Lbs) $C_{36}$-$C_{54}$ dimer/trimer fatty acid (Empol® 1022/Emery Chemicals)
The vessel was heated until the charge was molten. Agitation was started and the temperature adjusted to 80-85°C.

The Premier Mill was charged with 2mm glass beads to 60% of the available volume. The mill was preheated to 55°C by passing hot water through the mill chamber. Simultaneously, the mill shaft was started at 1100 RPM, lime slurry feed was started at 0.45 kg/min (1.01 lb/min.), and molten fatty acid feed was started at 0.34 kg/min (0.76 lb/min.) rate. The average retention time (ART) in the mill was 12.4 min. The temperature of the exiting material was controlled at 85°C by cooling water applied to the jacket of the mill. The product was cooled to room temperature by passing through a heat exchanger.

6

### Example 3

**Lime Slurry Feed**

339.75 kg (750 Lbs) Water
41.67 kg (92 Lbs) Hydrated Lime (MV300/Mississippi Lime Co.)
1.13 kg (2.5 Lbs) Ethoxylated Nonyl Phenol (Hyonic® PE-100/Henkel Corp.)
1.81 kg (4.0 Lbs) sodium salt of an Alkyl Aryl Sulfonate (Witconate® 1238/Witco)
21.74 kg (48 Lbs) Peg 600 Monolaurate (Nopalcol® 6-L/Henkel Corp.
Agitation was started and the temperature adjusted to 20-30°C.

**Fatty Acid Feed**

294.45 kg (650 Lbs) $C_{18}$ fatty acid (Industrene® 7018/Witco Chemical)
16.67 kg (37 Lbs) Ethoxylated Nonyl Phenol (Hyonic® PE-100/Henkel Corp.)
1.58 kg (3.5 Lbs) $C_{36}$-$C_{54}$ dimer/trimer fatty acid (Empol® 1022/Emery Chemicals)
The vessel was heated until the charge was molten. Agitation was started and the temperature adjusted to 80-85°C.

The Premier Mill was charged with 2mm glass beads to 60% of the available volume. The mill was preheated to 55°C by passing hot water through the mill chamber. Simultaneouly, the mill shaft was started at 1650 RPM, lime slurry feed was started at 0.90 kg/min (2.00 lb/min.), and molten fatty acid feed was started at 0.69 kg/min (1.53 lb/min.) rate. The average retention time (ART) in the mill was 5.2 min. The temperature of the exiting material was controlled at 85°C by cooling water applied to the jacket of the mill. The product was cooled to room temperature by passing through a heat exchanger.

### Example 4

**Lime Slurry Feed**

339.75 kg (750 Lbs) Water
41.67 kg (92 Lbs) Hydrated Lime (MV300/Mississippi Lime Co.)
1.13 kg (2.5 Lbs) Ethoxylated Nonyl Phenol (Hyonic® PE-100/Henkel Corp.)
1.81 kg (4.0 Lbs) sodium salt of an Alkyl Aryl Sulfonate (Witconate® 1238/Witco)
21.74 kg (48 Lbs) Peg 600 Monolaurate (Nopalcol® 6-L/Henkel Corp.
Agitation was started and the temperature adjusted to 20-30°C.

**Fatty Acid Feed**

294.45 kg (650 Lbs) $C_{18}$ fatty acid (Industrene® 7018/Witco Chemical)
16.67 kg (37 Lbs) Ethoxylated Nonyl Phenol (Hyonic® PE-100/Henkel Corp.)
1.58 kg (3.5 Lbs) $C_{36}$-$C_{54}$ dimer/trimer fatty acid (Empol® 1022/Emery Chemicals)
The vessel was heated until the charge was molten. Agitation was started and the temperature adjusted to 80-85°C.

The Premier Mill was charged with 2mm glass beads to 60% of the available volume. The mill was preheated to 55°C by passing hot water through the mill chamber. Simultaneously, the mill shaft was started at 1100 RPM, lime slurry feed was started at 0.95 kg/min (2.10 lb/min.), and molten fatty acid feed was started at 0.69 kg/min (1.53 lb/min.) rate. The average retention time (ART) in the mill was 6.1 min. The temperature of the exiting material was controlled at 55°C by cooling water applied to the jacket of the mill. The product was cooled to room temperature by passing through a heat exchanger.

### Example 5

**Lime Slurry Feed**

339.75 kg (750 Lbs) Water
41.67 kg (92 Lbs) Hydrated Lime (MV300/Mississippi Lime Co.)
1.13 kg (2.5 Lbs) Ethoxylated Nonyl Phenol (Hyonic® PE-100/Henkel Corp.)
1.81 kg (4.0 Lbs) sodium salt of an Alkyl Aryl Sulfonate (Witconate® 1238/Witco)

21.74 kg (48 Lbs) Peg 600 Monolaurate (Nopalcol® 6-L/Henkel Corp.)
Agitation was started and the temperature adjusted to 20-30°C.

Fatty Acid Feed

294.45 kg (650 Lbs) $C_{18}$ fatty acid (Industrene® 7018/Witco Chemical)
16.67 kg (37 Lbs) Ethoxylated Nonyl Phenol (Hyonic® PE-100/Henkel Corp.)
1.58 kg (3.5 Lbs) $C_{36}$-$C_{54}$ dimer/trimer fatty acid (Empol® 1022/Emery Chemicals)
The vessel was heated until the charge was molten. Agitation was started and the temperature adjusted to 80-85°C.

The Premier Mill was charged with 2mm glass beads to 60% of the available volume. The mill was preheated to 55°C by passing hot water through the mill chamber. Simultaneouly, the mill shaft was started at 1100 RPM, lime slurry feed was started at 1.62 kg/min (3.58 lb/min.), and molten fatty acid feed was started at 1.05 kg/min (2.32 lb/min.) rate. The average retention time (ART) in the mill was 3.6 min. The temperature of the exiting material was controlled at 80°C by cooling water applied to the jacket of the mill. The product was cooled to room temperature by passing through a heat exchanger.

Example 6

Lime Slurry Feed

339.75 kg (750 Lbs) Water
41.67 kg (92 Lbs) Hydrated Lime (MV300/Mississippi Lime Co.)
17.89 kg (39.5 Lbs) Ethoxylated Nonyl Phenol (Hyonic® PE-100/Henkel Corp.)
1.81 kg (4.0 Lbs) sodium salt of an Alkyl Aryl Sulfonate (Witconate® 1238/Witco)
21.74 kg (48 Lbs) Peg 600 Monolaurate (Nopalcol® 6-I/Henkel Corp.)
Agitation was started and the temperature adjusted to 20-30°C.

Fatty Acid Feed

294.45 kg (650 Lbs) $C_{18}$ fatty acid (Industrene® 7018/Witco Chemical)
1.58 kg (3.5 Lbs) $C_{36}$-$C_{54}$ dimer/trimer fatty acid (Empol® 1022/Emery Chemicals)
The vessel was heated until charge was molten. Agitation was started and the temperature adjusted to 80-85°C.

The Premier Mill was charged with 2mm glass beads to 60% of the available volume. The mill was preheated to 55°C by passing hot water through the mill chamber. Simultaneouly, the mill shaft was started at 1650 RPM, lime slurry feed was started at 1.37 kg/min (3.03 lb/min.), and molten fatty acid feed was started at 0.98 kg/min (2.16 lb/min.) rate. The average retention time (ART) in the mill was 4.0 min. The temperature of the exiting material was controlled at 60°C by cooling water applied to the jacket of the mill. The product was cooled to room temperature by passing through a heat exchanger.

Example 7

Lime Slurry Feed

328.42 kg (725 Lbs) Water
41.67 kg (92 Lbs) Hydrated Lime (MV300/Mississippi Lime Co.)
17.89 kg (39.5 Lbs) Ethoxylated Nonyl Phenol (Hyonic® PE-100/Henkel Corp.)
1.81 kg (4.0 Lbs) sodium salt of an Alkyl Aryl Sulfonate (Witconate® 1238/Witco)
10.19 kg (22.5 Lbs) Peg 600 Monolaurate (Nopalcol®6-L/Henkel Corp.)
Agitation was started and the temperature adjusted to 20-30°C.

Fatty Acid Feed

294.45 kg (650 Lbs) $C_{18}$ fatty acid (Industrene® 7018/Witco Chemical)
1.58 kg (3.5 Lbs) $C_{36}$-$C_{54}$ dimer/trimer fatty acid (Empol® 1022/Emery Chemicals)
The vessel was heated until the charge was molten. Agitation was started and the temperature adjusted to 80-85°C.

Additive Feed

9.97 kg (22 Lbs) Water
9.97 kg (22 Lbs) Peg 600 Monolaurate (Nopalcol 6-L/Henkel Corp.)

The Premier Mill was charged with 2mm glass beads to 60% of the available volume. The mill was preheated to 55°C by passing hot water through the mill chamber. Simultaneously, the mill shaft was started at 1650 RPM, lime slurry feed was started at 1.49 kg/min (3.30 lb/min.), and molten fatty acid feed was started at 0.98 kg/min (2.16 lb/min.) rate. The average retention time (ART) in the mill was 3.8 min. The temperature of the exiting material was controlled at 70°C by cooling water applied to the jacket of the mill. The product was Cooled to room temperature by passing through a heat exchanger. The product from the heat exchanger was post blended with the additive feed as follows:

43.94 kg (97 Lbs) of heat exchanger output
1.36 kg (3 Lbs) of the additive feed

The blending was done at 20-30°C.

Example 8

Lime Slurry Feed

253.68 kg (560 Lbs) Water
41.67 kg (92 Lbs) Hydrated Lime (MV300/Mississippi Lime Co.)
17.89 kg (39.5 Lbs) Ethoxylated Nonyl Phenol (Hyonic® PE-100/Henkel Corp.)
1.81 kg (4.0 Lbs) sodium salt of an Alkyl Aryl Sulfonate (Witconate® 1238/Witco)
10.19 kg (22.5 Lbs) Peg 600 Monolaurate (Nopalcol® 6-L/Henkel Corp.)

Agitation was started and the temperature adjusted to 20-30°C.

Fatty Acid Feed

294.45 kg (650 Lbs) $C_{18}$ fatty acid (Industrene® 7018/Witco Chemical)
1.58 kg (3.5 Lbs) $C_{36}$-$C_{54}$ dimer/trimer fatty acid (Empol® 1022/Emery Chemicals)

The vessel was heated until the charge was molten. Agitation was started and the temperature adjusted to 80-85°C.

The Premier Mill was charged with 2mm glass beads to 60% of the available volume. The mill was preheated to 55°C by passing hot water through the mill chamber. Simultaneouly, the mill shaft was started at 1650 RPM, lime slurry feed was started at 1.16 kg/min (2.56 lb/min.), and molten fatty acid feed was started at 0.98 kg/min (2.18 lb/min.) rate. The average retention time (ART) in the mill was 4.2 min. The temperature of the exiting material was controlled at 70°C by cooling water applied to the jacket of the mill. The product was cooled to room temperature by passing through a heat exchanger.

Example 9

Lime Slurry Feed

183.01 kg (404 Lbs) Water
31.25 kg (69 Lbs) Hydrated Lime (MV300/Mississippi Lime Co.)
12.45 kg (27.5 Lbs) ethoxylated nonyl phenol (Hyonic® PE-100/Henkel Corp.)
0.72 kg (1.6 Lbs) sodium salt of an Alkyl Aryl Sulfonate (Witconate® 1238/Witco)
2.26 kg (5.0 Lbs) Peg 600 Monolaurate (Nopalcol® 6-L/Henkel Corp.)

Agitation was started and the temperature adjusted to 20-30°C.

Fatty Acid Feed

201.13 kg (444 Lbs) $C_{18}$ fatty acid (Industrene® 7018/Witco Chemical)
1.04 kg (2.3 Lbs) $C_{36}$-$C_{54}$ dimer/trimer fatty acid (Empol® 1022/Emery Chemical)

The vessel was heated until the charge was molten, agitation was started and the temperature adjusted to 80-85°C.

Additive Feed

7.24 kg (16 Lbs) Water
13.59 kg (30 Lbs) Peg 600 Monolaurate (Nopalcol® 6-l/Henkel Corp.)
0.22 kg (0.5 Lbs) Benzisothiazolin-3-one (Proxel®GXL/ICI Americas)
The components were charged and mixed at 20-30°C.

The Premier Mill was charged with 2mm glass beads to 65% of the available volume. The mill was preheated to 55°C by passing hot water through the mill chamber. Simultaneouly, the mill shaft was started at 1650 RPM, lime slurry was started at 1.22 kg/min (2.70 lb/min.), and molten fatty acid feed was started at 1.14 kg/min (2.53 lb/min.) rate. The average retention time (ART) in the mill was 3.8 min. The temperature of the exiting material was controlled at 75°C by cooling water applied to the jacket of the mill. As the material exited the mill it was passed through a static mixer where 0.09 kg/min (0.20 lb/min.) of the additive feed was added. The product was then cooled to room temperature by passing through a heat exchanger.

Example 10

Lime Slurry Feed

185.73 kg (410 Lbs) Water
33.52 kg (74 Lbs) Hydrated Lime (MV300/Mississippi Lime Co.)
0.72 kg (1.6 Lbs) sodium salt of an Alkyl Aryl Sulfonate (Witconate® 1238/Witco)
2.35 kg (5.2 Lbs) Peg 600 Monolaurate (Nopalcol® 6-L/Henkel Corp.)
Agitation was started and temperature adjusted to 20-30°C.

Fatty Acid Feed

197.51 kg (436 Lbs) $C^{18}$ fatty acid (Industrene® 7018/Witco Chemical)
12.45 kg (27.5 Lbs) Ethoxylated Nonyl Phenol (Hyonic® PE-100/Henkel Corp.)
0.99 kg (2.2 Lbs) C36-C54 dimer/trimer fatty acid (Empol® 1022/Emery Chemical)
The vessel was heated until charge was molten, then agitation was started and temp adjusted to 80-85°C.

Additive Feed

7.25 kg (16 Lbs) Water
13.59 kg (30 Lbs) Peg 600 Monolaurate (Nopalcol® 6-L/Henkel Corp.)
0.22 kg (0.5 Lbs) Benzisothiazolin-3-One (Proxel® GXL/ICI Americas)
The components were charged and mixed at 20-30°C.

The Premier Mill was charged with 2mm glass beads to 65% of the available volume. The mill was preheated to 55°C by passing hot water thru the mill chamber. Simultaneouly, the mill shaft was started at 1650 RPM, lime slurry feed was started at 1.23 kg/min (2.71 lb/min.), and molten fatty acid feed was started at 1.15 kg/min (2.53 lb/min.) rate. The average retention time (ART) in the mill was 3.8 min. The temperature of the exiting material was controlled at 75°C by cooling water applied to the jacket of the mill. As the material exited the mill it was passed thru a static mixer where 0.11 kg/min (0.24 lb/min.) of the additive composition was added. The product was then cooled to room temperature by passing through a heat exchanger.

EXAMPLE 11

Lime Slurry Feed

284.03 kg (627 Lbs) Water
48.65 kg (107.4 Lbs) Hydrated Lime (MV300/Mississippi Lime Co.)
3.40 kg (7.5 Lbs) Peg 600 Monolaurate (Nopalcol® 6-L/Henkel Corp.)
Agitation was started and the temperature adjusted to 20-30°C.

Fatty Acid Feed

328.42 kg (725 Lbs) $C_{18}$ fatty acid (Industrene® 7018/Witco Chemical)
23.10 kg (51.0 Lbs) Ethoxylated Nonyl Phenol (Hyonic® PE-100/Henkel Corp.)
1.77 kg (3.9 Lbs) $C_{36}$-$C_{54}$ dimer/trimer fatty acid (Empol® 1022/Emery Chemicals)
1.22 kg (2.7 Lbs) sodium salt of an alkyl aryl sulfonate (Witconate® 1238/Witco)
7.02 kg (15.5 Lbs) Peg 600 Monolaurate (Nopalcol® 6-L/Henkel Corp.)
The vessel was heated until the charge was molten, agitation was started and the temperature adjusted to 80-85°C.

Additive Feed

25.46 kg (56.2 Lbs) Water
21.47 kg (47.4 Lbs) Peg 600 Monolaurate (Nopalcol® 6-L/Henkel Corp.)
0.32 kg (0.7 Lbs) Benzisothiazolin-3-one (Proxel® GXL/ICI Americas
The components were charged and mixed at 20-30°C.

The Premier Mill was charged with 2mm zirconium silicate beads to 90% of the available volume. the mill was preheated to 55°C by passing hot water through the mill chamber. Simultaneously, the mill shaft was started at 1650 RPM, lime slurry feed was started at 0.98 kg/min (2.16 lb/min.), and molten fatty acid feed was started at 1.07 kg/min (2.36 lb/min.) rate. The average retention time (ART) in the mill was 3.3 min. The temperature of the exiting material was controlled at 85°C by cooling water applied to the jacket of the mill. As the material exited the mill it was passed through a static mixer where 0.15 kg/min (0.33 lb/min.) of the additive feed was added. The product was cooled to room temperature by passing through a heat exchanger.

EXAMPLE 12

Lime Slurry Feed

284.03 kg (627 Lbs) Water
48.65 kg (107.4 Lbs) Hydrated Lime (MV300/Mississippi Lime Co.)
3.39 kg (7.5 Lbs) Peg 600 Monolaurate (Nopalcol® 6-L/Henkel Corp.)
Agitation was started and the temperature adjusted to 20-30°C.

Fatty Acid Feed

328.42 kg (725 Lbs) $C_{18}$ fatty acid (Industrene® 7018/Witco Chemical)
23.10 kg (51.0 Lbs) Ethoxylated Nonyl Phenol (Hyonic® PE-100/Henkel Corp.)
1.76 kg (3.9 Lbs) $C_{36}$-$C_{54}$ dimer/trimer fatty acid (Empol® 1022/Emery Chemicals)
1.22 kg (2.7 Lbs) sodium salt of an alkyl aryl sulfonate (Witconate® 1238/Witco)
7.02 kg (15.5 Lbs) Peg 600 Monolaurate (Nopalcol® 6-L/Henkel Corp.)
The vessel was heated until the charge was molten, agitation was started and the temperature adjusted to 80-85°C.

Additive Feed

25.45 kg (56.2 Lbs) Water
21.47 kg (47.4 Lbs) Peg 600 Monolaurate (Nopalcol® 6-L/Henkel Corp.)
0.31 kg (0.7 Lbs) Benzisothiazolin-3-one (Proxel® GXL/ICI Americas)
The components were charged and mixed at 20-30°C.

The Premier Mill was charged with 1.7 mm zirconium oxide beads to 80% of the available volume. The mill was preheated to 55°C by passing hot water through the mill chamber. Simultaneously, the mill shaft was started at 1650 RPM, lime slurry feed was started at 0.98 kg/min (2.18 lb/min.), and molten fatty acid feed was started at 1.05 kg/min (2.33 lb/min.) rate. The average retention time (ART) in the mill was 3.8 min. The temperature of the exiting material was controlled at 95°C by cooling water applied to the jacket of the mill. As the material exited the mill it was passed through a static mixer where 0.15 kg/min (0.33 lb/min.) of the additive feed was added. The product was cooled to room temperature by passing through a heat exchanger.

11

## EXAMPLE 13

### Lime Slurry Feed

245.52 kg (542 Lbs) Water
41.99 kg (92.7 Lbs) Hydrated Lime (MV300/Mississippi Lime Co.)
2.9 kg (6.5 Lbs) Peg 600 Monolaurate (Nopalcol® 6-L/Henkel Corp.)
Agitation was started and the temperature adjusted to 20-30°C.

### Fatty Acid Feed

293.77 kg (648.5 Lbs) $C_{18}$ fatty acid (Industrene® 7018/Witco Chemical)
20.65 kg (45.6 Lbs) Ethoxylated Non Phenol (Hyonic® PE-100/Henkel Corp.)
1.58 kg (3.5 Lbs) $C_{36}$-$C_{54}$ dimer/trimer fatty acid (Empol® 1022/Emery Chemicals)
1.13 kg (2.5 Lbs) sodium salt of an alkyl aryl sulfonate (Witconate® 1238/Witco)
The vessel was heated until the charge was molten, agitation was started and the temperature adjusted to 80-85°C.

### Additive Feed

29.35 kg (64.8 Lbs) Water
24.64 kg (54.4 Lbs) Peg 600 Monolaurate (Nopalcol® 6-L/Henkel Corp.)
0.36 kg (0.8 Lbs) Benzisothiazolin-3-one (Proxel® GXL/ICI Americas)
The components were charged and mixed at 20-30°C.

The Premier Mill was charged with 2mm zirconium silicate beads to 80% of the available volume. The mill was preheated to 55°C by passing hot water through the mill chamber. Simultaneously, the mill shaft was started at 1350 RPM, lime slurry feed was started at 0.98 kg/min (2.17 lb/min.), and molten fatty acid feed was started at 1.05 kg/min (2.32 lb/min.) rate. The average retention time (ART) in the mill was 3.8 min. The temperature of the exiting material was controlled at 85°C by cooling water applied to the jacket of the mill. As the material exited the mill, it was passed through a static mixer where 0.15 kg/min (0.33 lb/min.) of the additive feed was added. The product was cooled to room temperature by passing through a heat exchanger.

## EXAMPLE 14

### Lime Slurry Feed

245.52 kg (542 Lbs) Water
41.99 kg (92.7 Lbs) Hydrated Lime (MV300/Mississippi Lime Co.)
2.94 kg (6.5 Lbs) Peg 600 Monolaurate (Nopalcol® 6-L/Henkel Corp.)
Agitation was started and the temperature adjusted to 20-30°C.

### Fatty Acid Feed

293.77 kg (648.5 Lbs) $C_{18}$ fatty acid (Industrene® 7018/Witco Chemical)
20.65 kg (45.6 Lbs) Ethoxylated Non Phenol (Hyonic® PE-100/Henkel Corp.)
1.58 kg (3.5 Lbs) $C_{36}$-$C_{54}$ dimer/trimer fatty acid (Empol® 1022/Emery Chemicals)
1.13 kg (2.5 Lbs) sodium salt of an alkyl aryl sulfonate (Witconate® 1238/Witco)
7.02 kg (15.5 Lbs) Peg 600 Monolaurate (Nopalcol® 6-L/Henkel Corp.)
The vessel was heated until the charge was molten, agitation was started and the temperature adjusted to 80-85°C.

### Additive Feed

29.35 kg (64.8 Lbs) Water
24.64 kg (54.4 Lbs) Peg 600 Monolaurate (Nopalcol® 6-L/Henkel Corp.)
0.36 kg (0.8 Lbs) Benzisothiazolin-3-one (Proxel® GXL/ICI Americas)
The components were charged and mixed at 20-30°C.

The Premier Mill was charged with 1.5mm glass beads to 80% of the available volume. The mill was preheated to 55°C by passing hot water through the mill chamber. Simultaneously, the mill shaft was started at 1350 RPM, lime slurry feed was started at 0.978 kg/min (2.16 lb/min.), and molten fatty acid feed was started at 1.06 kg/min (2.36 lb/min.) rate. The average retention time (ART) in the mill was 3.8 min. The temperature of the exiting material was controlled at 95°C by cooling water applied to the jacket of the mill. As the material exited the mill, it was passed through a static mixer where 0.15 kg/min (0.33 lb/min.) of the composition in the addditives tank was added. The product was then cooled to room temp. by passing through a heat exchanger.

TABLE

| EXAMPLE | ART (MIN) | PROD. RATE kg/min (LB/MIN) | MILL OPERATING CONDITIONS | | | PRODUCT PROPERTIES | | | |
|---|---|---|---|---|---|---|---|---|---|
| | | | % BEAD LOAD | RPM | TEMP (°C) | % SOLIDS | VISCOSITY | ALK (% KOH) | % SCREEN |
| 1 | 8.9 | 0.78 (1.72) | 90 | 1650 | 70 | 47.7 | 360 CPS | 1.00 | 0.0000 |
| 2 | 12.4 | 0.80 (1.77) | 60 | 1100 | 85 | 50.0 | 275 CPS | 0.44 | 0.0013 |
| 3 | 5.2 | 1.60 (3.53) | 75 | 1650 | 85 | 49.5 | 350 CPS | 0.21 | 0.0002 |
| 4 | 6.1 | 1.64 (3.63) | 60 | 1100 | 55 | 50.5 | 750 CPS | 0.20 | 0.0120 |
| 5 | 3.6 | 2.67 (5.90) | 60 | 1100 | 80 | 48.1 | 975 CPS | 1.90 | 0.0060 |
| 6 | 4.0 | 2.35 (5.19) | 60 | 1650 | 60 | 49.0 | 625 CPS | 0.65 | N/A |
| 7 | 3.8 | 2.47 (5.46) | 60 | 1650 | 70 | N/A | 150 CPS | 1.20 | 0.0020 |
| 8 | 4.2 | 2.13 (4.72) | 60 | 1650 | 70 | 55.3 | 395 CPS | 1.51 | 0.0040 |
| 9 | 4.2 | 2.48 (5.48) | 55 | 1650 | 75 | 55.3 | 270 CPS | 1.02 | 0.0040 |
| 10 | 3.8 | 2.48 (5.48) | 65 | 1650 | 75 | 55.5 | 355 CPS | 0.36 | 0.0030 |
| 11 | 3.3 | 2.20 (4.86) | 90% ZIR SIL | 1650 | 85 | 55.5 | 290 CPS | 0.33 | 0.0026 |
| 12 | 3.8 | 2.19 (4.84) | 80% ZIR OX | 1650 | 95 | 56.5 | 150 CPS | 0.19 | 0.0040 |
| 13 | 3.8 | 2.18 (4.82) | 80% ZIR SIL | 1350 | 85 | 56.2 | 270 CPS | 0.37 | 0.0030 |
| 14 | 3.8 | 2.19 (4.85) | 80% GLASS | 1350 | 95 | 55.2 | 305 CPS | 0.18 | 0.0030 |

ALK-ALKALINITY EXPRESSED AS %KOH, MEASURED 1-24 HRS AFTER REACTION
% SCREEN - % OF METAL SOAP RETAINED ON A 0.044mm (325 mesh) SCREEN
ART-AVERAGE RETENTION TIME MINUTES
* ZIR SIL = ZIRCONIUM SILICATE BEADS
ZIR OX = ZIRCONIUM OXIDE BEADS
ALL OTHER BEADS ARE GLASS

ALK = ALKALINITY EXPRESSED AS % KOH, MEASURED 1 - 24 HRS AFTER COMPLETION

% SCREEN= RETENTION ON A 0.044mm (325 MESH) SCREEN (DAY BASIS)

13

**Claims**

1. A continuous process for preparing an aqueous dispersion of a metal soap which comprises: forming a mixture by continuously introducing at least one carboxylic acid having from 6 to 30 carbon atoms, at least one oxide or hydroxide of a metal, which forms a metal soap with the at least one carboxylic acid, water and at least one dispersing agent into a media mill, wherein water comprises at least 25% by weight of the mixture and reacting the mixture in the media mill for from 1 to 55 minutes to form an aqueous dispersion of the metal soap which comprises from 25% to 65% water.

2. A process of claim 1 wherein the at least one carboxylic acid is selected from the group consisting of 10 to 22 carbon atom monocarboxylic acids.

3. A process of claim 1 wherein the at least one oxide or hydroxide of a metal is selected from the group consisting of oxides and hydroxides of aluminum, baruim, calcium, cadmium, cobalt, copper, iron, lead, lithium, magnesium, magnese, nickel, strontium and zinc.

4. A process of claim 1 wherein the reaction is carried out at a temperature of from 25°C to 100°C.

5. A process of claim 1 wherein the water, at least a portion of the dispersing agent and the oxide or hydroxide of the metal are introduced into the media mill as a mixture.

6. A process of claim 1 wherein at least a portion of the dispersing agent is introduced into the media mill admixed with the carboxylic acid.

7. A process of claim 1 which comprises: forming a feed mixture comprising the oxide or hydroxide of the metal, water and at least a portion of the dispersing agent; continuously introducing the feed mixture and molten carboxylic acid into the media mill to form the mixture; and reacting the mixture in the media mill at a temperature from 35°C to 95°C for from 1 to 20 minutes to form the aqueous dispersion of the metal soap.

8. A process of claim 7 wherein a portion of the dispersing agent is mixed with the carboxylic acid before the carboxylic acid is introduced into the media mill.

9. A process of claim 8 wherein the dispersing agent comprises from 0.5% to 20% by weight of the mixture.

10. A process of claim 1 wherein the acid is stearic acid and the metal hydroxide is calcium hydroxide.

11. A process of claim 1 wherein the temperature of the material in the media mill is below the melting point of the carboxylic acid.

12. A process of claim 1 wherein the media mill is a horizontal stirred media mill.

13. A process of claim 12 which comprises;
    a) forming a mixture of water, dispersing agent and metal oxide or hydroxide;
    b) forming a mixture of a fatty acid having from 8 to 30 carbon atoms and dispersing agent at a temperature at which the mixture is molten;
    c) continuously introducing a) and b) into the media mill, maintained at a temperature of from 35°C to 95°C and reacting a) and b) for from 1 to 20 minutes; and
    d) recovering as aqueous metal soap dispersion.

14. A process of claim 13 wherein the temperature in the media mill is below the melting point of the fatty acid dispersion agent mixture.

15. A process of claim 13 wherein the temperature in the media mill is above the melting point of the fatty acid dispersion agent mixture.

**16.** A process of Claim 1 wherein the aqueous dispersion of metal soap comprises from 30% to 55% water.

**Patentansprüche**

**1.** Kontinuierliches Verfahren zur Herstellung einer wäßrigen Dispersion einer Metallseife, umfassend: Bildung einer Mischung durch kontinuierliches Einführen wenigstens einer Carbonsäure mit 6 bis 30 Kohlenstoffatomen, wenigstens eines Oxids oder Hydroxids eines Metalls, welches eine Metallseife mit der wenigstens einen Carbonsäure bildet, von Wasser und wenigstens eines Dispersionsmittels in eine Perlmühle ("media mill"), worin Wasser wenigstens 25 Gew.-% der Mischung umfaßt, und Umsetzung der Mischung in der Perlmühle während 1 bis 55 Minuten unter Bildung einer wäßrigen Dispersion der Metallseife, die 25 % bis 65 % Wasser umfaßt.

**2.** Verfahren gemäß Anspruch 1, worin die wenigstens eine Carbonsäure aus der Gruppe, bestehend aus Monocarbonsäuren mit 10 bis 22 Kohlenstoffatomen, ausgewählt ist.

**3.** Verfahren gemäß Anspruch 1, worin das wenigstens eine Oxid oder Hydroxid eines Metalls aus der Gruppe, bestehend aus Oxiden und Hydroxiden des Aluminiums, Bariums, Calciums, Cadmiums, Cobalts, Kupfers, Eisens, Bleis, Lithiums, Magnesiums, Mangans, Nickels, Strontiums und Zinks, ausgewählt ist.

**4.** Verfahren gemäß Anspruch 1, worin die Reaktion bei einer Temperatur von 25 °C bis 100 °C durchgeführt wird.

**5.** Verfahren gemäß Anspruch 1, worin das Wasser, wenigstens ein Teil des Dispersionsmittels und das Oxid oder Hydroxid des Metalls in die Perlmühle als eine Mischung eingeführt werden.

**6.** Verfahren gemäß Anspruch 1, worin wenigstens ein Teil des Dispersionsmittels in die Perlmühle, vermischt mit einer Carbonsäure, eingeführt wird.

**7.** Verfahren gemäß Anspruch 1, umfassend: Bildung der Zugabemischung, die das Oxid oder Hydroxid des Metalls, Wasser und wenigstens einen Teil des Dispersionsmittels umfaßt, kontinuierliches Einführen der Zugabemischung und der geschmolzenen Carbonsäure in die Perlmühle, um die Mischung zu bilden und Umsetzung der Mischung in der Perlmühle bei einer Temperatur von 35 °C bis 95 °C während 1 bis 20 Minuten, um die wäßrige Dispersion der Metallseife zu bilden.

**8.** Verfahren gemäß Anspruch 7, worin ein Teil des Dispersionsmittels mit der Carbonsäure vor dem Einführen der Carbonsäure in die Perlmühle vermischt wird.

**9.** Verfahren gemäß Anspruch 8, worin das Dispersionsmittel 0,5 Gew.-% bis 20 Gew.-% der Mischung umfaßt.

**10.** Verfahren gemäß Anspruch 1, worin die Säure Stearinsäure ist und das Metallhydroxid Calciumhydroxid ist.

**11.** Verfahren gemäß Anspruch 1, worin die Temperatur des Materials in der Perlmühle unterhalb des Schmelzpunkts der Carbonsäure liegt.

**12.** Verfahren gemäß Anspruch 1, worin die Permühle eine horizontal gerührte Perlmühle ist.

**13.** Verfahren gemäß Anspruch 12, umfassend:
a) Bildung einer Mischung aus Wasser, Dispersionsmittel und Metalloxid oder -hydroxid,
b) Bildung einer Mischung aus einer Fettsäure mit 8 bis 30 Kohlenstoffatomen und Dispersionsmittel bei einer Temperatur, bei der die Mischung geschmolzen ist,
c) kontinuierliches Einführen von a) und b) in die Perlmühle, die bei einer Temperatur von 35 °C bis 95 °C gehalten wird, und Umsetzen von a) und b) während 1 bis 20 Minuten und
d) Gewinnung als wäßrige Metallseifen-Dispersion.

**14.** Verfahren gemäß Anspruch 13, worin die Temperatur in der Perlmühle unterhalb des Schmelzpunkts der Fettsäure/Dispersionsmittel-Mischung liegt.

**15.** Verfahren gemäß Anspruch 13, worin die Temperatur in der Perlmühle oberhalb des Schmelzpunkts der Fettsäure/Dispersionsmittel-Mischung liegt.

**16.** Verfahren gemäß Anspruch 1, worin die wäßrige Dispersion der Metallseife 30 Gew.-% bis 55 Gew.-% Wasser umfaßt.

**Revendications**

**1.** Procédé continu de préparation d'une dispersion aqueuse d'un savon métallique, qui comprend : la formation d'un mélange par introduction continue d'au moins un acide carboxylique ayant de 6 à 30 atomes de carbone, d'au moins un oxyde ou hydroxyde d'un métal, qui forme un savon métallique avec le ou les acides carboxyliques, de l'eau et d'au moins un agent dispersant dans un broyeur à garniture, dans lequel l'eau constitue au moins 25 % du poids du mélange, et la réaction du mélange dans le broyeur à garniture pendant 1 à 55 minutes pour former une dispersion aqueuse du savon métallique qui comprend de 25 % à 65 % d'eau.

**2.** Procédé selon la revendication 1, dans lequel le ou les acides carboxyliques est/sont choisi(s) dans le groupe constitué par les acides monocarboxyliques de 10 à 22 atomes de carbone.

**3.** Procédé selon la revendication 1, dans lequel le ou les oxydes ou hydroxydes d'un métal est/sont choisi(s) dans le groupe constitué par les oxydes et hydroxydes d'aluminium, de baryum, de calcium, de cadmium, de cobalt, de cuivre, de fer, de plomb, de lithium, de magnésium, de manganèse, de nickel, de strontium et de zinc.

**4.** Procédé selon la revendication 1, dans lequel la réaction est effectuée à une température de 25°C à 100°C.

**5.** Procédé selon la revendication 1, dans lequel l'eau, au moins une portion de l'agent dispersant et l'oxyde ou hydroxyde de métal sont introduits dans le broyeur à garniture sous forme d'un mélange.

**6.** Procédé selon la revendication 1, dans lequel au moins une portion de l'agent dispersant est introduite dans le broyeur à garniture mélangée avec l'acide carboxylique.

**7.** Procédé selon la revendication 1, qui comprend : la formation d'un mélange d'alimentation comprenant l'oxyde ou l'hydroxyde de métal, l'eau et au moins une portion de l'agent dispersant ; l'introduction continue du mélange d'alimentation et d'acide carboxylique fondu dans le broyeur à garniture, pour former le mélange ; et la réaction du mélange dans le broyeur à garniture à une température de 35°C à 95°C pendant 1 à 20 minutes, pour former la dispersion aqueuse du savon métallique.

**8.** Procédé selon la revendication 7, dans lequel une portion de l'agent dispersant est mélangée avec l'acide carboxylique avant que l'acide carboxylique soit introduit dans le broyeur à garniture.

**9.** Procédé selon la revendication 8, dans lequel l'agent dispersant constitue de 0,5 % à 20 % du poids du mélange.

**10.** Procédé selon la revendication 1, dans lequel l'acide est l'acide stéarique et l'hydroxyde métallique est l'hydroxyde de calcium.

**11.** Procédé selon la revendication 1, dans lequel la température de la matière dans le broyeur à garniture est inférieure au point de fusion de l'acide carboxylique.

**12.** Procédé selon la revendication 1, dans lequel le broyeur à garniture est un broyeur à garniture agité horizontal.

**13.** Procédé selon la revendication 12 qui comprend :

a) la formation d'un mélange d'eau, d'agent dispersant et d'oxyde ou d'hydroxyde métallique ;

b) la formation d'un mélange d'un acide gras ayant de 8 à 30 atomes de carbone et d'agent dispersant, à une température à laquelle le mélange est fondu ;

c) l'introduction continue de a) et b) dans le broyeur à garniture maintenu à une température de 35 °C à 95 °C et la réaction de a) et b) pendant 1 à 20 minutes ; et

d) la récupération d'une dispersion aqueuse de savon métallique.

14. Procédé selon la revendication 13, dans lequel la température du broyeur à garniture est inférieure au point de fusion du mélange d'acide gras et d'agent dispersant.

15. Procédé selon la revendication 13, dans lequel la température du broyeur à garniture est supérieure au point de fusion du mélange d'acide gras et d'agent dispersant.

16. Procédé selon la revendication 1, dans lequel la dispersion aqueuse de savon métallique comprend 30 % à 55 % d'eau.

FATTY
ACID

FIG. 1

PRIOR ART

FIG. 2